Europäisches Patentamt

European Patent Office

Office européen des brevets

(11) Veröffentlichungsnummer: **0 243 310**
**A2**

## EUROPÄISCHE PATENTANMELDUNG

(21) Anmeldenummer: **87810232.6**

(22) Anmeldetag: **10.04.87**

(51) Int.Cl.³: **G 01 N 33/36**

(30) Priorität: **18.04.86 CH 1568/86**
**03.02.87 CH 385/87**

(43) Veröffentlichungstag der Anmeldung:
**28.10.87 Patentblatt 87/44**

(84) Benannte Vertragsstaaten:
**BE CH DE ES FR GB IT LI**

(71) Anmelder: **CIBA-GEIGY AG**
**Klybeckstrasse 141**
**CH-4002 Basel(CH)**

(72) Erfinder: **Gisin, Markus, Dr.**
**Steinweg 5**
**CH-4147 Aesch(CH)**

(72) Erfinder: **Thommen, Christian**
**Roggenburgstrasse 29**
**CH-4055 Basel(CH)**

(54) Verfahren zur Steuerung und Optimierung industrieller Prozesse.

(57) Beschrieben wird eine Verfahren zur Steuerung und Optimierung chemischer, industrieller Prozese, das dadurch gekennzeichnet ist, dass man die Methode der Strömungs-Injektions-Analyse zur Steuerung und Optimierung von Herstellungsverfahren von Textilveredlungsmitteln und Textilausrüstmitteln und deren Zwischenprodukten anwendet.

Vorteil dieses Verfahrens ist, dass quasi der Istzustand eines Verfahrens ständig gemessen, beurteilt und zur feedback bzw. feed-forward Steuerung verwendet werden kann.

Fig. 1

EP 0 243 310 A2

CIBA-GEIGY AG

Basel (Schweiz)

1-15841/1+2/=/ZFO

## Verfahren zur Steuerung und Optimierung industrieller Prozesse

Die vorliegende Erfindung betrifft ein neues Verfahren zur Steuerung und Optimierung industrieller Prozesse, indem man die Methode der Strömungs- (oder Fliess-)Injektions-Analyse (flow injection analysis) auf Herstellungsverfahren für Textilveredlungsmittel und Textilausrüstmittel und deren Zwischenprodukte anwendet.

In den letzten Jahren ist man zunehmend bestrebt, Herstellungsverfahren für Textilveredlungsmittel und Textilausrüstmittel und deren Zwischenprodukte, wie z.B. Farbstoffe, Aufheller und deren Zwischenprodukte, zu automatisieren und zu optimieren, und zwar sowohl was den Herstellungsprozess anbetrifft, als auch hinsichtlich der Aufarbeitung. Um hier zu befriedigenden und reproduzierbaren Ergebnissen zu gelangen, ist man auf Analysenmethoden angewiesen, die sich durch folgende Kriterien auszeichnen: kurze Analysendauer, hohe Analysenfrequenz bzw. hoher Informationsgehalt pro Zeiteinheit, möglichst geringe Kosten, Einfachheit, Zuverlässigkeit und geringer Platzbedarf. Geräte, die diesen Anforderungen genügen, sind vor allem Sensoren für die Messung physikalischer Parameter, wie z.B. Temperatur, Druck und Verbrauch an Ausgangsmaterialien.

Diese Parameter liefern jedoch keine Information über den augenblicklichen Verfahrensstand und Verfahrenszustand (Ausbeute an gewünschtem Produkt, Anteil an Nebenprodukten, Produktqualität) und den jeweiligen Verlauf der chemischen Reaktion. Damit sind Kontrolle und Sicherstellung einer gleichbleibenden Produktqualität verbunden mit optimaler Ausbeute nicht gewährleistet. Ferner sind aus den physikalischen Parametern keine Prognosen hinsichtlich Reaktionsende, Produktqualität und Ausbeute möglich. Es ist bisher üblich,
die Reaktionsmasse am Ende einer Verfahrensstufe oder erst am Ende
des ganzen Verfahrens zu analysieren; damit sind korrigierende
Massnahmen im Verlauf des Verfahrens ausgeschlossen.

Sowohl für kontinuierliche wie insbesondere für diskontinuierliche
Herstellungsverfahren für Textilveredlungsmittel und Textilausrüstmittel und deren Zwischenprodukte wird heute unabhängig von der
Qualität der Ausgangsmaterialien eine gleichbleibende Qualität der
Verfahrensprodukte bei optimaler Ausbeute verlangt.

Insbesondere diskontinuierliche Herstellungsverfahren (sog. batch-
Prozesse) führen leicht zu schwankenden Qualitäten der Verfahrensprodukte bedingt durch unterschiedliche Qualität der Ausgangsverbindungen, und die Ausbeute ist von Partie zu Partie verschieden.

Aufgabe der Erfindung ist es daher, eine Analysenmethode zu finden,
die den genannten Anforderungen entspricht, und ein Verfahren zur
Steuerung und Optimierung der Herstellungsverfahren für Textilveredlungsmittel und Textilausrüstmittel und deren Zwischenprodukte
mit dieser Analysenmethode zu entwickeln, so dass Steuerung und
Optimierung insbesondere auch im on-line Betrieb durchgeführt werden
können.

Gefunden wurde, dass die Strömungs-Injektions-Analysen-Technik
hervorragend für diesen Zweck geeignet ist.

Die Strömungs-Injektions-Analyse (flow injection analysis) ist eine
nasschemische Automationstechnik basierend auf kontinuierlichen
Reagenzienströmen in Kapillarrohren, der reproduzierbaren Probeneinführung durch Injektion, der kontrollierten Dispersion der
Probenzone auf ihrem Weg zum Detektor sowie einem präzisen zeitlichen Ablauf einer Einzelanalyse. Ein Strömungs-Injektions-Analysator zeichnet sich aus durch eine kurze Ansprechzeit, hohe Bei den Farbstoffen, deren Synthese und Verarbeitung mittels der hier beschriebenen Methode überwacht und gesteuert wird, handelt es sich in erster Linie um Textilfarbstoffe, die den verschiedensten chemischen Klassen angehören. Es handelt sich beispielsweise um anionische Farbstoffe, wie Nitro-, Aminoketon-, Ketonimin-, Methin-, Nitrodiphenylamin-, Chinolin-, Aminonaphthochinon- oder Cumarinfarbstoffe oder auch saure Farbstoffe auf Basis von Gelbholzextrakt, insbesondere saure Anthrachinon- und Azofarbstoffe, wie Monoazo- und Disazofarbstoffe. Ferner kommen basische, d.h. kationische Farbstoffe in Frage. Es handelt sich hier beispielsweise um die Halogenide, Sulfate, Methosulfate oder Metallhalogenid-Salze, z.B. Tetrachlorzinkate von Azofarbstoffen, wie Monoazo-, Disazo-und Polyazofarbstoffen, von Anthrachinonfarbstoffen, Phthalocyaninfarbstoffen; Diphenylmethan- und Triarylmethanfarbstoffen, Methin-, Polymethin- und Azomethinfarbstoffen, von Thiazol-, Ketonimin-, Acridin-, Cyanin-, Nitro-, Chinolin-, Benzimidazol-, Xanthen-, Azin-, Oxazin- und Thiazinfarbstoffen.

Bevorzugte Farbstoffe, deren Synthese und Verarbeitung mittels der hier beschriebenen Methode überwacht und gesteuert wird, sind saure Farbstoffe der Formel

$$D\text{---}(X)_m$$

worin D der Rest eines organischen Farbstoffes der Monoazo- oder Polyazo-, Metallkomplexazo-, Anthrachinon-, Phthalocyanin-, Formazan-, Azomethin-, Nitroaryl-, Dioxazin-, Phenazin- oder Stilbenreihe, X ein faserreaktiver Rest, der direkt oder über ein Brückenglied an den Rest D gebunden ist, und $m = 0, 1, 2, 3$ oder 4 ist.

Als Farbstoffe kommen insbesondere solche in Betracht wie z.B. in der EP-A-0 178 255 auf den Seiten 6 bis 8 oder der EP-A-0 177 449 auf den Seiten 13 bis 18 angegeben.

Unter dem Begriff Farbstoffe sollen im vorliegenden Fall auch optische Aufheller verstanden werden, z.B. Stilbenaufheller, vor allem solche vom Typ der Bis-triazinylaminostilben-disulfonsäuren, der Bis-styrylbiphenyle und -benzole und der Bis-triazolylstilbendisulfonsäuren.

Es versteht sich von selbst, dass je nach Aufgabenstellung im Zuge der Farbstoffsynthese neben den eigentlichen Farbstoffen bzw. Aufhellern, nach dem erfindungsgemässen Verfahren auch Vor- und Nebenprodukte erfasst werden. Gerade die während der Reaktion gebildeten Nebenprodukte sind von grosser Bedeutung, nicht nur hinsichtlich der Ausbeute, sondern auch was den Farbton bzw. die Eigenfarbe des entsprechenden Farbstoffs/Aufhellers anbetrifft.

Grundsätzlich ist das Verfahren von grosser Anwendungsbreite und lässt sich in der Farbstoffherstellung zur Steuerung und Regelung der Farbstoffsynthese, zur Steueurng und Regelung von Edukt- und Produktflüssen zur Analyse und zur Qualitätskontrolle einsetzen.

Die Methode der Strömungs-Injektions-Analyse bietet die Möglichkeit, eine nahezu Echtzeitsteuerung oder direkte Steuerung eines Verfahrens [near-real-time monitoring] vorzunehmen. Dies beruht auf den folgenden Eigenschaften der Strömungs-Injektions-Analyse:
- sequentielle Durchführung von Einzelanalysen im kontinuierlichen Flüssigkeitsstrom;
- kurzer Zeitbedarf pro Einzelanalyse, ca. 30 Sekunden, d.h. kurze Ansprechzeit des Analysators;
- hohe Injektionsfrequenz, z.B. mehr als 100 pro Stunde;
- gute und schnelle Regeneration des Systems, wobei die Basislinie unmittelbar vor und unmittelbar nach jeder Einzelanalyse den Zustand des Analysators anzeigt.

Diese Eigenschaften der Strömungs-Injektions-Analyse definieren ein Modulations-prinzip, das einen physikalisch-chemischen oder chemischen Ursprung hat.

0243310

Injektionsfrequenz, eine genaue Kenntnis des Systemzustands vor und unmittelbar nach der Einzelanalyse (Modulation).

Die Strömungs-Injektions-Analyse ist als solche bekannt, verwiesen wird auf folgende Literatur: E.H. Hansen und J. Ruzicka "Flow Injection Analysis", John Wiley & Sons, New York, 1981; C.B. Ranger in "Automated Stream Analysis for Process Control", Band 1,. Seite 39 ff, D.P. Manka, Akademic Press 1982.

Das bisher primäre Anwendungsgebiet der Strömungs-Injektions-Analyse ist die Automation nass-chemischer Analysenmethoden im Laboratorium. Ferner ist die Strömungs-Injektions-Analysen-Technik zur Ueberwachung einiger weniger kontinuierlicher chemischer Verfahren angewendet worden.

Gegenstand der vorliegenden Erfindung ist ein Verfahren zur Steuerung und Optimierung chemischer, industrieller Prozesse, welches dadurch gekennzeichnet ist, dass man die Methode der Strömungs-Injektions-Analyse zur Steuerung und Optimierung von Herstellungsverfahren von Textilveredlungsmitteln und Textilausrüstmitteln und deren Zwischenprodukten anwendet.

Eine bevorzugte Ausführungsform des erfindungsgemässen Verfahrens ist dadurch gekennzeichnet, dass man die Methode der Strömungs-Injektions-Analyse zur Steuerung und Optimierung diskontinuierlicher Herstellungsverfahren (batch-Prozesse) anwendet.

Der Strömungs-Injektions-Analysator, der erfindungsgemäss verwendet werden kann, besteht beispielsweise aus einem Vorratsbehälter für eine Carrier-Lösung, z.B. Wasser, einer handelsüblichen Pumpe, einem üblichen Probeninjektionssystem, welches ein reproduzierbares Probevolumen, beispielsweise aus einem by-pass Probenahme-System, in den Strom der Carrier-Lösung einspritzt, einer Kapillare geeigneter Länge oder einer gerührten Mischkammer zur Verdünnung der Probe im Carrier-Strom und zur Bildung eines Konzentrationsgradienten, und einem Detektor, beispielsweise einem Durchflussphotometer.

Ueberraschenderweise kann mit dem erfindungsgemässen Verfahren nicht nur der augenblickliche Verfahrensstand, wie z.B. bisher erzielte Ausbeute an Endprodukt, Anteil an Nebenprodukten festgestellt und überwacht werden, sondern der Prozess kann bedingt durch die grosse Anzahl prozesstechnisch relevanter Daten pro Zeiteinheit, die sich aus der Strömungs-Injektions-Analysen-Technik ergibt, auf eine gewünschte Qualität (Verfahrensproduktezusammensetzung) hin gesteuert werden, indem die im Detektor ermittelten Daten zur Prozesssteuerung verwendet werden (feed-back, bzw. insbesondere feed-forward Steuerung). Ferner ergibt sich aus jeder neuen Analyse eine Prognose über den weiteren Verfahrensablauf und das Verfahrensende.

Durch Messung beispielsweise spektraler Daten während der Reaktion werden folgende Informationen gewonnen und zur Prozesssteuerung verwendet:
- optimaler Zeitpunkt für die Beendigung der Reaktion,
- Ausbeutekontrolle,
- Kontrolle der Reaktionsbedingungen, indem Abweichungen sich beispielsweise durch vermehrte Bildung von Nebenprodukten ausdrücken,
- Qualitätskontrolle durch Bestimmung bzw. Festsetzung der Nebenprodukte.

Textilveredlungsmittel sind beispielsweise Farbstoffe und optische Aufheller. Textilausrüstmittel sind beispielsweise Weichmachungsmittel, Zusätze zum Flammfestausrüsten oder schmutz-, wasser- und öl-abweisende Mittel.

Ganz besonders bevorzugt wendet man das erfindungsgemässe Verfahren auf Herstellungsverfahren für Farbstoffe, Aufheller und deren Zwischenprodukte an.

Das vom Detektor aufgezeichnete Signal (transient) enthält einen instrumentellen Beitrag, die Basislinie, und den Mess-Beitrag. Im Gegensatz zu Analysatoren, welche direkt im kontinuierlichen Probestrom messen, gestattet die Strömungs-Injektions-Analysen-Methode zwischen instrumentellem Beitrag, d.h. die Aenderung der Basislinie, und dem Mess-Beitrag zweifelsfrei zu unterscheiden.

Die oben genannten Eigenschaften sind von grosser Bedeutung für die Verwendung der Strömungs-Injektions-Analyse als Teil eines computergesteuerten Herstellungsverfahrens für Textilveredlungsmittel und Textilausrüstmittel und deren Zwischenprodukte.

Die Strömungs-Injektions-Analyse bietet verschiedene Möglichkeiten, Daten zur Prozesssteuerung zu liefern.

Neben der prozesstechnisch wichtigen Bestimmung von Standardparametern, wie z.B. Edukt-, Produkt- und Nebenproduktkonzentration bietet sich die Methode der Strömungs-Injektions-Analyse an zur gezielten, reproduzierbaren Festsetzung des Verfahrensendpunktes sowie zur Prognose des weiteren zeitlichen Verfahrensablaufes und der Verfahrensqualität (Anteil an Edukt, Produkt und Nebenprodukt).

Eine ganz besonders bevorzugte Ausführungsform des erfindungsgemässen Verfahrens ist dadurch gekennzeichnet, dass man mit der Strömungs-Injektions-Analyse bzw. mit den daraus ermittelten Daten ein chemisches Verfahren auf eine vorgegebene Qualität (Endpunkt des Verfahrens) hinsteuert. Diese mittels Strömungs-Injektions-Analyse vorgenommene Prozesssteuerung kann z.B. durch gleichzeitige Signalhöhen- und Signalbreiten-Messung der im Detektor, z.B. Durchflussphotometer, registrierten Daten erfolgen. Diese Messmethode ist besonders zur Kontrolle und Steuerung diskontinuierlicher Verfahren geeignet, in deren Verlauf Edukt- und Produktkonzentrationen sehr stark schwanken (z.B. vom Kilo-Mol bis zum mMol Bereich). Bei dieser Mess-Methode ist es nicht notwendig die Probenzone zu verdünnen. Bei der Signalbreiten-Messung wird die Zeitdifferenz zwischen zwei identischen Regionen der Probenzone (Anfang und Ende der Zone) gemessen, wobei die Identität der Regionen der Probenzone sich auf die Verdünnung und/oder chemische Umsetzung bezieht. Grosse Konzentrationsunterschiede sind während des Verfahrens mit der Methode der Signalbreiten-Messung gut feststellbar. Die Signalhöhen-Messung ist eine empfindlichere und präzisere Messmethode, die sich insbesondere zur Steuerung im kritischen Bereich eines Vefahrens eignet.

Die Steuerung eines chemischen Verfahrens mittels Strömungs-Injektions-Analyse auf eine vorgegebene Qualität hin erfolgt z.B. durch Signalhöhen- und Signalbreiten-Messung, wobei erstere für den kritischen Bereich des Verfahrens und letztere für den weniger kritischen Bereich angewendet werden kann.

Die Höhe des aufgezeichneten Signals bei der Signalhöhen-Messung ist im unteren Konzentrationsbereich proportional zur Konzentration. Bei höheren Konzentrationen verläuft die Eichkurve zunehmend flacher bis das System chemisch gesättigt ist. Es ist daher nicht möglich, anhand von z.B. filtrierten Proben eines diskontinuierlichen Verfahrens den gesamten Reaktionsverlauf mittels Signalhöhen-Messung zu verfolgen. Durch Auswertung der Signalbreite können hohe Konzentrationen mit dem gleichen System wie für die Endpunkt-Bestimmung gemessen werden. Die Signalbreite ist annähernd proportional zum Logarithmus der Konzentration der Probe. Grundsätzlich kann die Signalbreite auf beliebiger Höhe über der Basislinie gemessen werden. Infolge des logarithmischen Zusammenhanges zwischen Signalbreite und Konzentration ist die Präzision dieser Konzentrationsbestimmung geringer als bei der Signalhöhen-Messung. Der Uebergang der Signalbreiten-Messung zu der Signalhöhen-Messung erfolgt, sobald die Konzentration der gemessenen Probe den linearen Bereich der Signalhöhen-Messung des Strömungs-Injektions-Systems erreicht.

Ferner kann anstelle einer Kapillare zur Ausbildung des Konzentrationsgradienten auch eine gerührte Mischkammer verwendet werden, wodurch ein anderes Konzentrationsprofil gebildet wird, die Eichgerade der Signalbreiten-Messung steiler ist und damit die Präzision besser ist.

Anstelle der Auswertung der durch Signalbreiten-Messung erhaltenen Daten kann auch die sogenannte elektronische Verdünnung [electronic dilution] zur Erweiterung des dynamischen Messbereichs angewendet werden. Anstelle der Messung nur beim Signalmaximum werden bei dieser Methode Daten z.B. Extinctionswerte an verschiedenen zeitlich fixierten Orten in der absteigenden Flanke des Gradienten gemessen. Jeder dieser Messorte deckt einen limitierten Konzentrationsbereich der Probe mit einer linearen Eichgeraden ab. Die Präzision der Messung ist am Signalmaximum am grössten und nimmt mit zunehmender Entfernung vom Signalmaximum ab. Für die Steuerung eines Herstellungsverfahrens vor Erreichen des Endpunktes genügen im allgemeinen Messwerte mit geringerer Präzision.

Bei Verwendung einer Mischkammer anstelle einer Kapillare wird aufgrund der besser reproduzierbaren Verdünnung eine bessere Präzision erhalten.

Eine interessante Verfahrensweise mittels der Strömungs-Injektions-Analyse ein Verfahren auf den Endpunkt hin zu steuern, ist dadurch gekennzeichnet, dass man die Konzentration eines Gases in einer Flüssigkeit bestimmt. Diese Verfahrensweise wird so ausgeführt, dass die ein Gas enthaltende Reaktionslösung z.B. an einer Membran vorbeigeleitet wird, so dass das Gas aus der Reaktionslösung heraus durch diese Membran in einen Aufnehmerstrom und letzterer in den Strömungs-Injektions-Analysator eingespeist werden kann und dort gegebenenfalls nach Umsetzungen zu einer vom Detektor messbaren Verbindung registriert wird, und die erhaltenen Daten zur Steuerung verwendet werden. Eine weitere Verfahrensweise ist dadurch gekennzeichnet, dass eine Probe der Reaktionslösung über ein Ventil in eine Carrier-Lösung eingespeist wird, und dieser Strom an einer Membran vorbeigeleitet wird, sodass Gas aus der Carrier-Lösung heraus durch diese Membran in eine zweite Carrier-Lösung mit den Nachweisreagenzien eingespeist werden kann. Als Gase kommen beispielsweise in Betracht: Amine, wie Methylamin, Dimethylamin, $NH_3$, Stickstoffoxide, HCl, HCN, $H_2S$, $O_2$, $O_3$, Halogene.

Ein typisches Beispiel für die Anwendung dieser Verfahrensweise bei der Farbstoffherstellung ist die Diazotierung eines Amins. In Bezug auf den Herstellungsprozess einer der wichtigsten Farbstoffklassen, der Azofarbstoffe, besteht nach wie vor ein Bedarf an einfachen Methoden zur Ueberwachung eines wesentlichen Syntheseschrittes, nämlich der Diazotierungsreaktion. Eine Kontrolle der Diazotierung ist über die Erfassung der Distickstofftrioxid-Konzentration möglich. Bei der Diazotierung, die üblicherweise mit Natriumnitrit und Salzsäure vorgenommen wird, bildet sich über die Stufe der salpetrigen Säure Distickstofftrioxid, welches mit dem Amin zu der Diazonium-Verbindung umgesetzt wird. Bei der Diazotierung wird man über die Intensität des registrierten Signals zweckmässigerweise die Nitrit- oder Aminzufuhr steuern, d.h. wenn das Signal einen durch Vorversuche ermittelten Sollwert erreicht hat, wird die Nitrit- bzw. Aminzufuhr entsprechend korrigiert. Auf diese Weise kann man eine Ueberdosierung an Nitrit oder Amin vermeiden und auch während der Reaktion die Nitritzufuhr so steuern, dass es zu keinen unerwünschten Nebenreaktionen kommt.

Für die Steuerung der Diazotierung ist eine empfindliche Analysenmethode von Vorteil. Das in der Reaktionslösung vorhandene Distickstofftrioxid, welches durch die Membran in den zweiten Carrierstrom des Analysators diffundiert, reagiert mit einem in der Carrier-Lösung vorhandenem Amin zur Diazonium-Verbindung, der dann eine Kupplungskomponente zugesetzt wird. Die entsprechend der Konzentration des Distickstoffdioxids mehr oder weniger stark gefärbte Carrier-Lösung wird im Detektor z.B. einem Durchflussphotometer analysiert. Wenn in der Reaktionslösung alles Amin diazotiert ist, steigt bei weiterer Zugabe an Nitrit die Konzentration des Distickstofftrioxids an, was einen Intensitätsanstieg des registrierten Signals zur Folge hat. Wenn der durch Eichung ermittelte Sollwert erreicht wird, wird die Zufuhr an Nitrit gestoppt, d.h. die mit Hilfe des Spektrophotometers gemessenen Intensitätsschwankungen werden von einem Prozessrechner verarbeitet, der nach Vergleich mit dem Sollwert über ein entsprechendes Signal die Reaktandenströme
steuert.

Die Zufuhr an Nitrit kann auch portionsweise erfolgen, wobei die
Geschwindigkeit der Konzentrations-Abnahme registriert wird.
Aufgrund dieser Messungen kann der Endpunkt vorausgesagt werden.

Eine ebenfalls ganz besonders bevorzugte Ausführungsform des
erfindungsgemässen Verfahrens ist dadurch gekennzeichnet, dass man
mit der Strömungs-Injektions-Analyse Edukt-, Produkt- und Nebenprodukt-Konzentrationen gleichzeitig bestimmt. Bei stark unterschiedlichen Konzentrationen hat sich die Mischkammer als geeignet
erwiesen, wodurch präzise Gradienten-Formation über einen grossen
Konzentrationsbereich ermöglicht wird, und Matrix-Effekte (z.B.
Lösungsmitteleffekte) eliminiert werden. Die Eliminierung von
Matrix-Effekten ist notwendig bei Verfahren, in deren Verlauf sich
beispielsweise die Zusammensetzung des Lösungsmittels kontinuierlich
und drastisch ändert. Ein exponentieller Gradient bietet sich für
die Verdünnung an, wodurch präzise Messungen über einen grossen
Bereich ermöglicht werden. Ferner eignet sich die Mischkammer für
chemische Verfahren, bei welchen zum Zeitpunkt der Probennahme bzw.
der Messung die Weiterreaktion dieser Probe gestoppt werden muss,
z.B. durch Abkühlung, wozu sich die Verdünnungsmethode besonders
eignet, oder deren Reaktionsprodukte unterschiedlich gute Löslichkeit in der Trägerflüssigkeit (Carrier-Medium) haben, so dass durch
Verdünnung ein Auskristallisieren vermieden wird.

Die Verwendung einer Mischkammer mindert zwar die Analysenfrequenz,
wichtig für die Prozesssteuerung ist jedoch nicht nur die Analysenfrequenz sondern auch die Anzahl an Information pro Zeiteinheit bzw.
Analyse.

Im Zusammenhang mit der Mischkammer kann in dem erfindungsgemässen
Verfahren eine weitere Methode zur Erweiterung des dynamischen
Messbereichs von Strömungs-Injektions-Systemen angewendet werden. Es
handelt sich um die Gradienten-Verdünnung kombiniert mit zeitlich variablem "Einfangen" einer Probe [zone sampling]. Durch diesen im Analysator integrierten variablen Verdünnungsschritt kann ein nahezu beliebiger Messbereich erfasst werden. Wenn eine Probe in eine gerührte Mischkammer eingespritzt wird, erhält man beim Mischkammerausgang einen exponentiell abnehmenden Konzentrationsgradienten. Dieser Gradient weist alle Konzentrationen zwischen der kleinstmöglichen und der grösstmöglichen Verdünnung auf. Durch ein Ventil kann man den Teil des Gradienten, welcher der gewünschten Verdünnung entspricht "einfangen" [zone sampling] und in ein zweites Strömungs-Injektions-System einspritzen. Auf diese Weise sind Verdünnungen bis zu 1:100000 mit sehr guter Präzision (besser als 1 % relativer Standardabweichung) leicht durchführbar.

Bei konstanter Fliessgeschwindigkeit hängt die Verdünnung der Probe einzig von dem Zeitpunkt des "Einfangens" ab. Dies ermöglicht die Steuerung der Probeverdünnung aufgrund der aktuellen Probekonzentration per Computer.

Vorteile dieser Technik sind, dass
- störende Substanzen verdünnt werden und
- die Probe im System konditioniert werden kann.

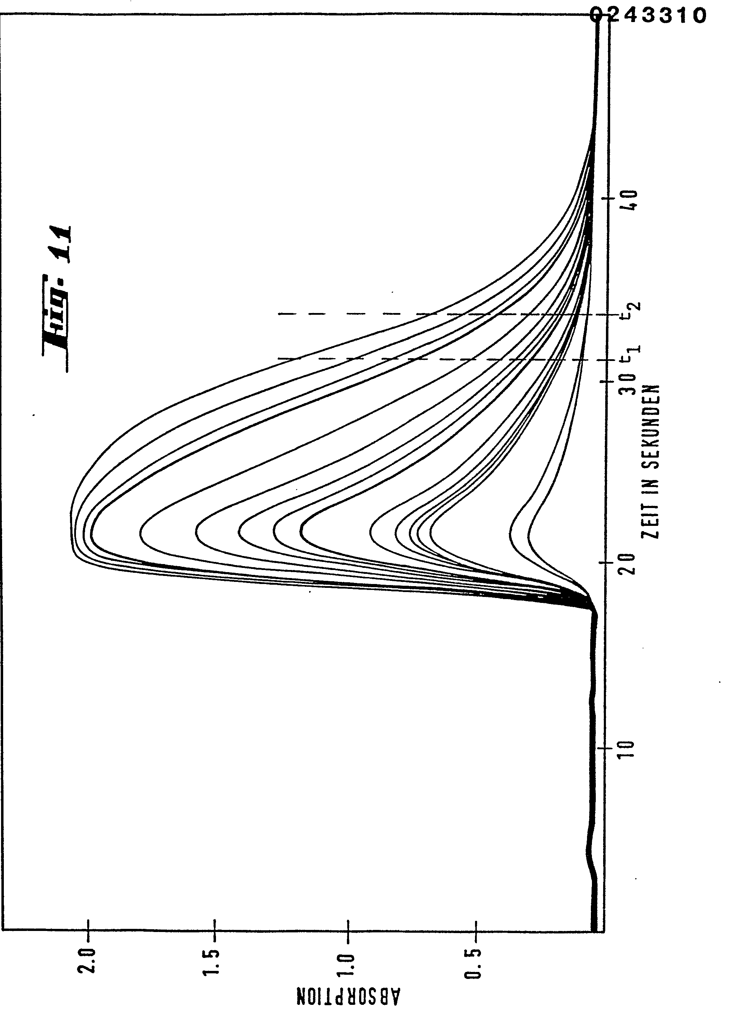

Figur 11 zeigt schematisch die oben beschriebene Methode. Die sich während der Reaktion ständig ändernde Konzentration der Edukte und Produkte wird in Figur 11 durch die verschiedenen Gradienten dargestellt. Die Gradienten entsprechen der Konzentration der Probe nach Verlassen der Mischkammer. Jede Probe, die in Abhängigkeit vom augenblicklichen Verfahrenszustand einem der angegebenen Gradienten nach Verlassen der Mischkammer entspricht, wird zum Zeitpunkt $t_1$ als ein Segment des Gradienten, welches durch den Abschnitt $t_1$ und $t_2$ symbolisiert ist, über ein Ventil in das zweite Strömungs-Injektions-System eingespeist und analysiert. Der Zeitpunkt $t_1$ wird der jeweiligen Konzentration angepasst.

Eine weitere ganz bevorzugte Ausführungsform des erfindungsgemässen Verfahrens ist dadurch gekennzeichnet, dass man die Steuerung eines chemischen Verfahrens mittels der umgekehrten Strömungs-Injektions-Analyse [reversed flow injection analysis] durchführt, wobei in zeitlich fixierten Abständen Reaktionslösung in den Analysator eingespritzt wird, diese Lösung verdünnt wird und gegebenenfalls vorbehandelt wird, wie z.B. durch Zusatz eines Puffersystems, und anschliessend in diese verdünnte Probenlösung einer der aktiven Reaktionspartner (z.B. ein Diazoniumsalz in die Lösung einer Kupplungskomponente) eingespritzt wird, wodurch im Detektor ein konzentrationsabhängiges Signal registriert wird. Die zur Steuerung des Verfahren relevanten Daten können nach einer der oben angegebenen Methoden ausgewertet werden.

Grundsätzlich ist das erfindungsgemässe Verfahren von grosser Anwendungsbreite und lässt sich bei der Herstellung insbesondere von Farbstoffen, optischen Aufhellern und deren Vor- bzw. Zwischenprodukten, zur Steuerung und Regelung der Synthese, zur Steuerung und Regelung von Edukt-, Produkt- und Nebenproduktflüssen zur Analyse und zur Qualitätskontrolle einsetzen.

So lassen sich z.B. Einphasen- oder Multiphasen-Herstellungsverfahren steuern und optimieren. Insbesondere wendet man die Methode der Strömungs-Injektions-Analyse zur on-line-Steuerung an, vorzugsweise zur on-line-Steuerung eines computer-integrierten, automatisierten Herstellungsverfahrens für Textilveredlungsmittel und Textilausrüstmittel und deren Zwischenprodukte, z.B. Farbstoffe, optische Aufheller und deren Zwischenprodukte.

Eine bevorzugte Verfahrensweise zur Steuerung und Optimierung chemischer industrieller Prozesse ist dadurch gekennzeichnet, dass man die Methode der Strömungs-Injektions-Analyse zur Steuerung und Optimierung von Herstellungsverfahren von Textilveredlungsmitteln, Textilausrüstmitteln und deren Zwischenprodukten anwendet, wobei das Verfahren folgende von einem Rechner gesteuerte Schritte aufweist:
(1) Probenahme, gegebenenfalls mit Konditionierung der Probe;

(2) Injektion der Probe in einen gegebenenfalls Reagenzien enthaltenden Carrier-Strom;

(3) Transport eines oder mehrerer Carrier-Ströme mittels einer oder mehrerer Pumpen durch eine Kapillare geeigneter Länge oder einer gerührten Mischkammer zur Verdünnung der Probe im Carrier-Strom und zur Ausbildung eines Konzentrationsgradienten;

(4) Transport des Carrier-Stroms zu einem Detektor (z.B. Durchflussphotometer);

(5) Messung geeigneter Werte im Detektor;

(6) Uebertragung der gemessenen Werte zum Rechner;

(7) Vergleich der gemessenen Werte mit einem Sollwert; sowie

(8) rechnergesteuerte Einflussnahme auf das Verfahren (z.B. Steuerung der Reaktanden-Ströme).

Ein wesentliches Merkmal des erfindungsgemässen Verfahrens zur Steuerung und Optimierung von Textilveredlungsmitteln und Textilausrüstmitteln und deren Zwischenprodukten, insbesondere von Farbstoffen und optischen Aufhellern und deren Zwischenprodukten, ist das Probenahme-System, denn es handelt sich vorzugsweise um Dispersionen [slurries], die konditioniert werden müssen, bevor sie in das Strömungs-Injektions-System eingespeist werden. Diese Konditionierung kann aus einem Lösungs- und Verdünnungsschritt bestehen, so dass nur gelöste Teile eingespeist werden. Vorzugsweise wird in dem erfindungsgemässen Verfahren ein by-pass Probenahme-System mit

- einer kontinuierlichen Filtration;
- einer Homogenisierung und/oder Verdünnung der Probe mit einem Lösungsmittel; oder
- einer Membran z.B. einer Teflonmembran, wodurch messtechnisch relevante Teile der Probe diffundieren können,

angewendet.

Durch geeignete Wahl des Probenahmesystems lassen sich bereits bei der Probenahme Stoffe, welche die Analyse stören, abscheiden.

Das erfindungsgemässe Verfahren zur Steuerung und Optimierung von Textilveredlungsmitteln und Textilausrüstmitteln und deren Zwischenprodukten, insbesondere Farbstoffe und optische Aufheller und deren Zwischenprodukte, ist insbesondere dadurch gekennzeichnet, dass man die Methode der Strömungs-Injektions-Analyse zur Steuerung und Optimierung anwendet, wobei die zur Steuerung und Optimierung notwendigen Proben über ein by-pass Probenahme-System mit kontinuierlicher Filtration oder mit einer Homogenisierung oder Verdünnung der Probe mit einem Lösungsmittel, um ungelöste Anteile zu konditionieren bzw. zu lösen, oder mit einer Membran konditioniert werden, so dass nur gelöste Anteile in das Strömungs-Injektions-System eingespeist werden und zur Abdeckung eines grossen Konzentrationsbereichs, wie bei diskontinuierlicher Herstellungsweise der Textilveredlungsmittel, Textilausrüstmittel und deren Zwischenprodukten notwendig, Daten über Signalhöhen-Messung, Signalbreiten-Messung oder elektronischer Verdünnung oder Gradientenverdünnung mit variablem zone-sampling im System erfasst und verarbeitet werden.

Weitere wesentliche Merkmale des erfindungsgemässen Verfahrens betreffen das Injektionssystem, für das sich Rotationsventile als besonders geeignet erwiesen haben, da diese eine hohe Präzision gewährleisten, sowie die Pumpen zum Transport der Lösungen, für die sich z.B. Spritzenpumpen wie z.B. Motorkolbenbüretten als vorteilhaft erwiesen haben, da diese pulsationsfrei arbeiten, eine konstante Flussrate gewährleisten und exakt ansteuerbar sind sowie organische und aggressive Reagenzien pumpen können.

Ein spezieller Vorteil der Strömungs-Injektions-Analysen-Technik ist die problemlose Verwendung von z.B. instabilen Nachweisreagenzien, wie z.B. Diazonium-Ionen, die Selbstreinigung des Systems, die hohe Messfrequenz, welche die feed-forward-Steuerung ermöglicht.

Eine bevorzugte Ausführungsform des erfindungsgemässen Verfahrens ist dadurch gekennzeichnet, dass man zur Steuerung und Optimierung von Herstellungsverfahren von Textilveredlungsmitteln, Textilausrüstmitteln und deren Zwischenprodukten, insbesondere Farbstoffe,
optische Aufheller und deren Zwischenprodukte, die Methode der
Strömungs-Injektions-Analyse anwendet, wobei der Analysator aus

- einem Probenahmesystem,
- einem Injektionssystem,
- einem oder mehreren Carrier-Strömen, welche gegebenenfalls
  Reagenzien enthalten,
- einer oder mehrerer Spritzenpumpen zum Transport der Ströme,
- einer oder mehrerer Kapillaren oder einer gerührten Mischkammer,
  und
- einem Detektor

besteht, wobei das Probenahmesystem insbesondere entweder aus einem
Ventil im Fall von vollständig gelösten Proben oder einem Ventil mit
vorgeschalteter kontinuierlicher Filtration, Homogenisierung
und/oder Verdünnung der Probe mit einem Lösungsmittel oder einer
Membran besteht, wodurch nur messtechnisch relevante Teile der Probe
bzw. nur gelöste Teile der Probe in das Analysator-System eingespeist werden.

Das Pumpensystem besteht vorzugsweise aus einer Spritzenpumpe
wie z.B. einer Motorkolbenbürette.

Vorteile der Strömungs-Injektions-Analysen-Technik bei der on-line
Steuerung gegenüber Mess-Sonden in einer by-pass-Leitung oder in
einem Reaktionsgefäss sind die Trennung des analytischen Signals von
der Basislinie sowie die einfache Möglichkeit der Nachkalibration
während des laufenden Verfahrens. Durch die Kontrolle der Basislinie
können z.B. Verschmutzungen des Strömungs-Injektions-Systems früh
erkannt und z.B. durch Einschalten eines Spülcycluses sofort ohne
Einfluss auf das laufende Verfahren und dessen Steuerung als
Gegenmassnahme eingeleitet werden.

Bevorzugte Verfahrensweisen sind dadurch gekennzeichnet, dass man
- eine gerührte Mischkammer zur Ausbildung des Konzentrationsgradienten im Strömungs-Injektions-Analysator verwendet.

- die Methode der Strömungs-Injektions-Analyse zur Steuerung und
  Optimierung von Acylierungsreaktionen verwendet. Unter Acylierung
  wird die Kondensation eines faserreaktiven oder nichtfaserreaktiven Acylrestes mit einem Amin, einer Hydroxygruppe oder
  einer Thiolgruppe verstanden.

- die Methode der Strömungs-Injektions-Analyse zur Steuerung und
  Optimierung von Diazotierungen verwendet.

- zur Steuerung und Optimierung von Diazotierungsreaktionen eine
  Gasdiffusionszelle als Probenahmesystem oder Probenaufbereitungssystem verwendet.

- die Methode der Strömungs-Injektions-Analyse zur Steuerung und
  Optimierung von Kupplungsreaktionen verwendet.

- eine Probe, die mittels kontinuierlicher Filtration, Homogenisierung oder Verdünnung mit einem Lösungsmittel oder mittels einer
  Membran konditioniert wurde, im Strömungs-Injektions-Analysator
  verwendet.

Beschreibung der Figuren:
Figur 1 zeigt schematisch den Aufbau eines Strömungs-Injektions-
Analysators, wobei $R_1$, $R_2$, $R_3$ und C je ein Reservoir für Reagenzienlösungen, S der Probestrom, P je eine Pumpe, $L_1$, $L_2$, $L_3$ und $L_4$ je
eine Kapillare zur Vermischung der Reagenzienströme, V ein Einspritzventil und D ein Durchflussphotometer ist.

Figur 2 zeit den Verlauf einer mittels Strömungs-Injektions-Analyse
gesteuerten Acylierung bis zum Endpunkt (E), wobei die Signalfolge
übereinander gezeichnet wurde.

Figur 3 zeigt die Antwort eines Strömungs-Injektions-Analysators auf
dreimaligen Zusatz ($A_1$, $A_2$ und $A_3$) von Acylierungsmittel zu der
Reaktionsmasse nahe beim Endpunkt der Umsetzung, wobei H den Daten
der Signalhöhenmessung und W den Daten der Signalbreitenmessung entspricht und B die Basislinie darstellt. Um beide Auswertmethoden nebeneinander in einer Abbildung darzustellen, wurden für die Signalbreiten-Messung (W) die Werte einer Zeitskala auf Durchlässigkeitswerte umskaliert.

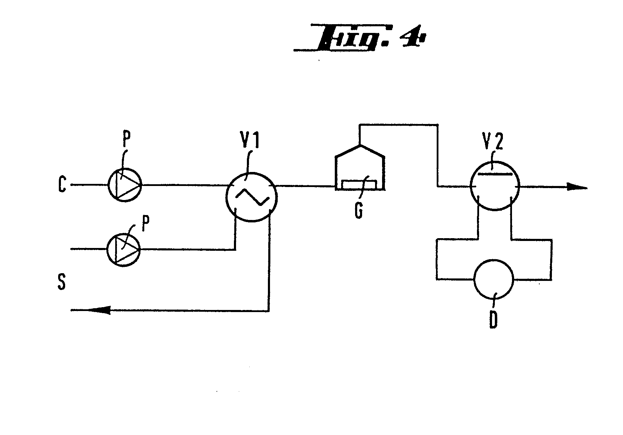

Figur 4 zeigt den Aufbau eines Strömungs-Injektions-Analysators, wobei C ein Carrier-Strom, S der Probenstrom, P je eine Pumpe, $V_1$ und $V_2$ je ein Einspritzventil, G eine magnetisch gerührte Mischkammer und D ein Durchflussphotometer ist.

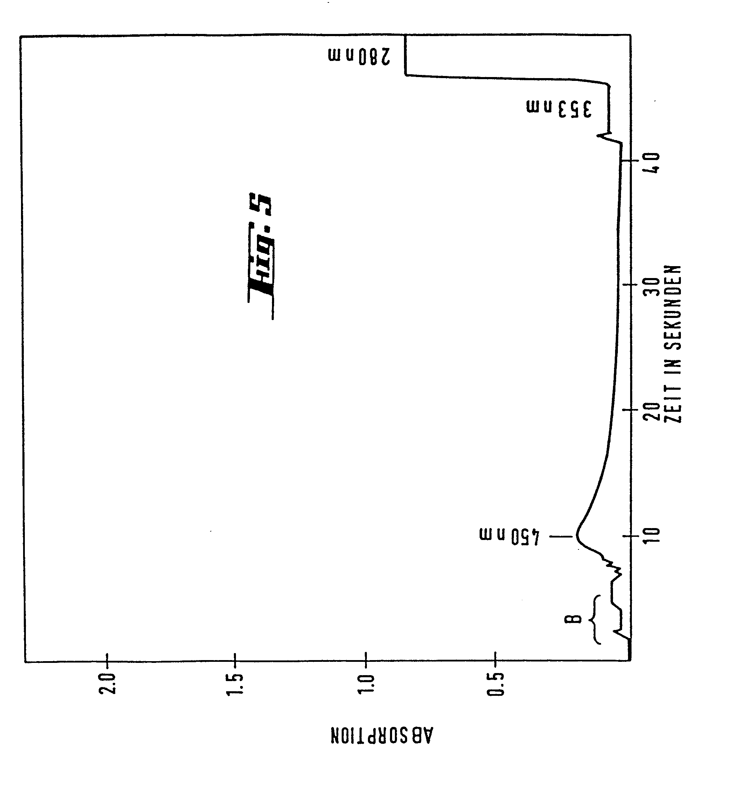

Figur 5 zeigt den Verlauf der mittels Strömungs-Injektions-Analyse gesteuerten Herstellung von Dinitrostilbendisulfonsäure am Anfang der Umsetzung, wobei B die Basislinienadsorption ist.

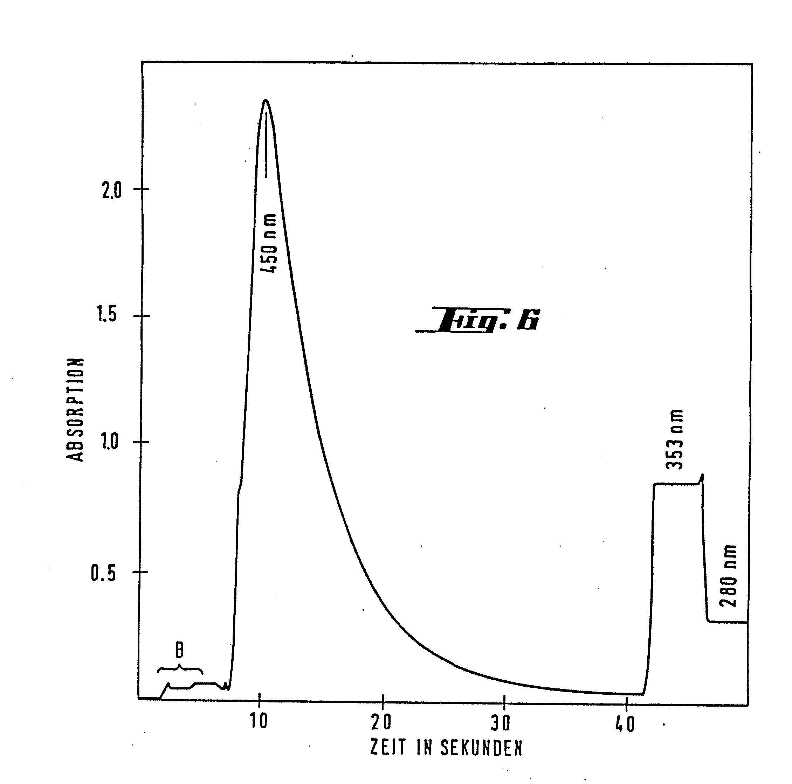

Figur 6 zeigt die Endphase der mittels Strömungs-Injektions-Analyse gesteuerten Herstellung von Dinitrostilbendisulfonsäure, wobei B die Basislinienadsorption ist.

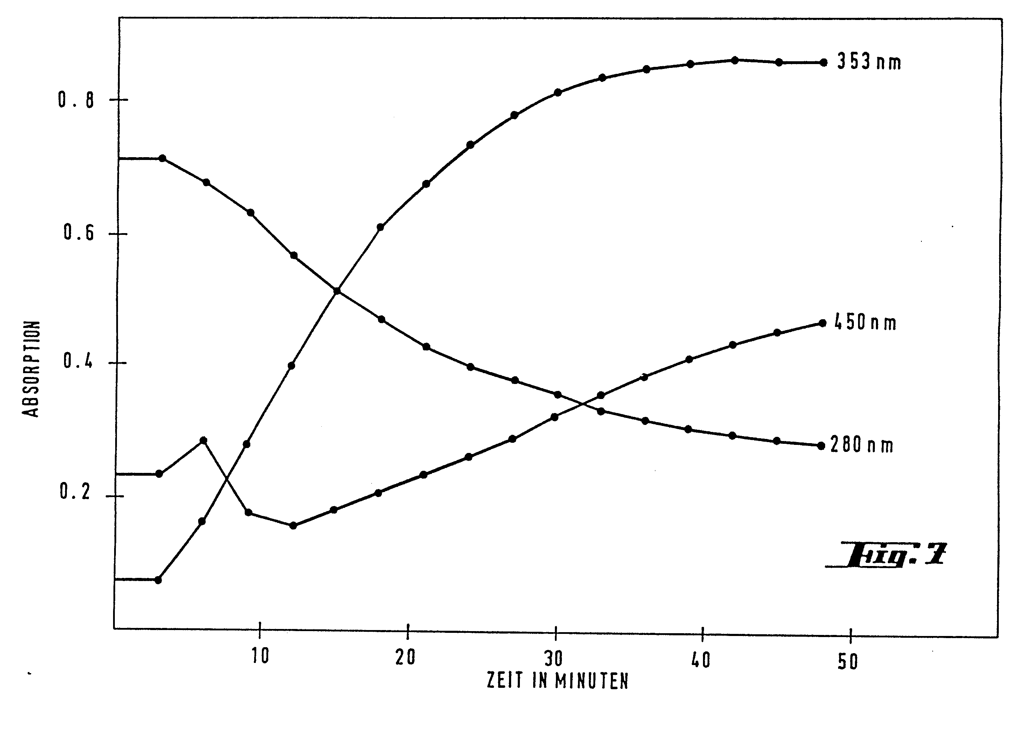

Figur 7 zeigt die mittels Strömungs-Injektions-Analyse gesteuerte Herstellung von Dinitrostilbendisulfonsäure für die gesamte Umsetzung, wobei die Absorptionswerte basislinienkorrigiert sind.

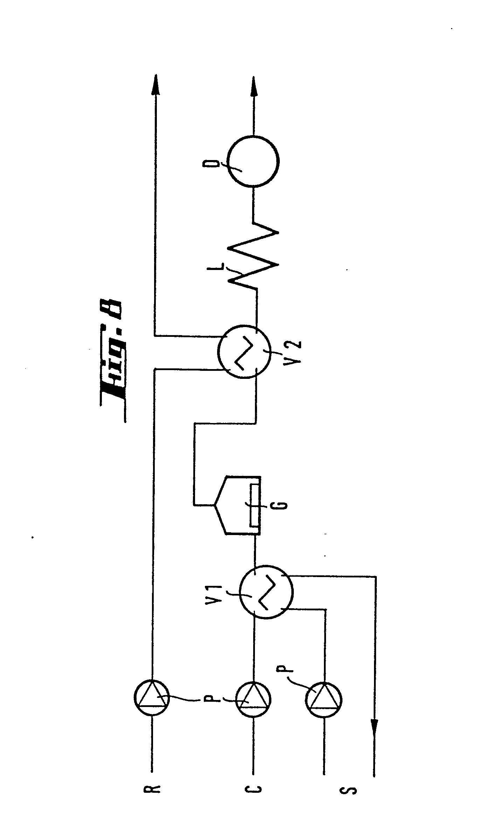

Figur 8 zeigt den Aufbau eines Analysators zur Messung der umgekehrten Strömungs-Injektions-Analyse, wobei R ein Reagenzienstrom, C ein Carrier-Strom, S der Probenstrom, P je eine Pumpe, $V_1$ das Probeeinspritzventil, $V_2$ das Reagenzeinspritzventil, G eine magnetisch gerührte Mischkammer, L eine Säule und D ein Durchflussphotometer ist.

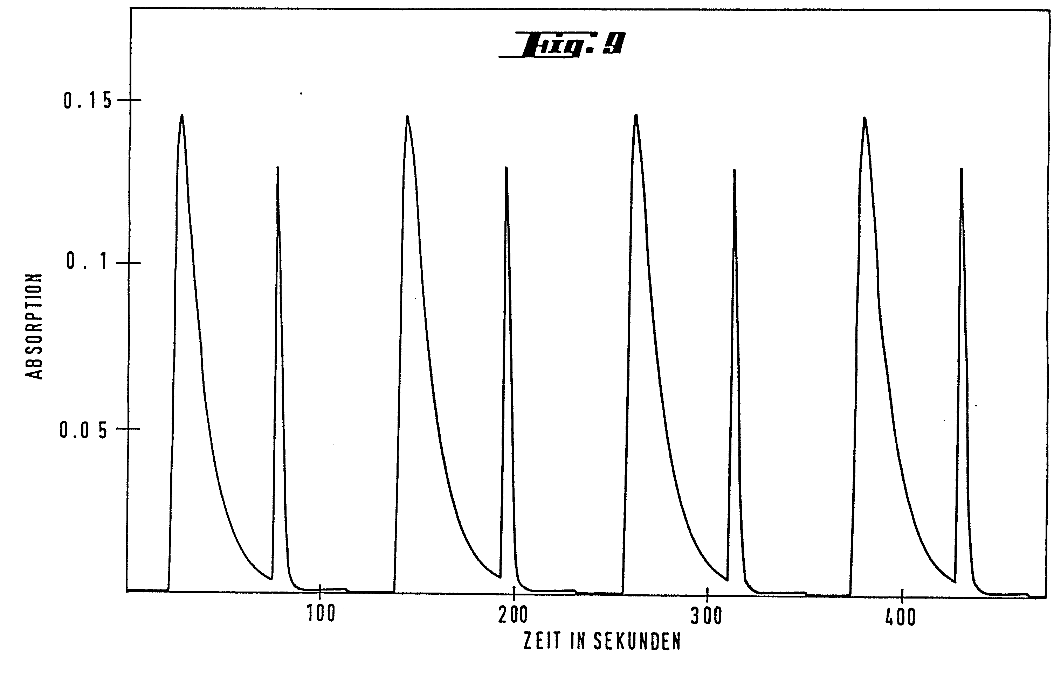

Figur 9 zeigt die Reproduktion von vier umgekehrten Strömungs-Injektion in den Gradienten der Probe bei einem Verdünnungskoeffizienten von ca. 3000.

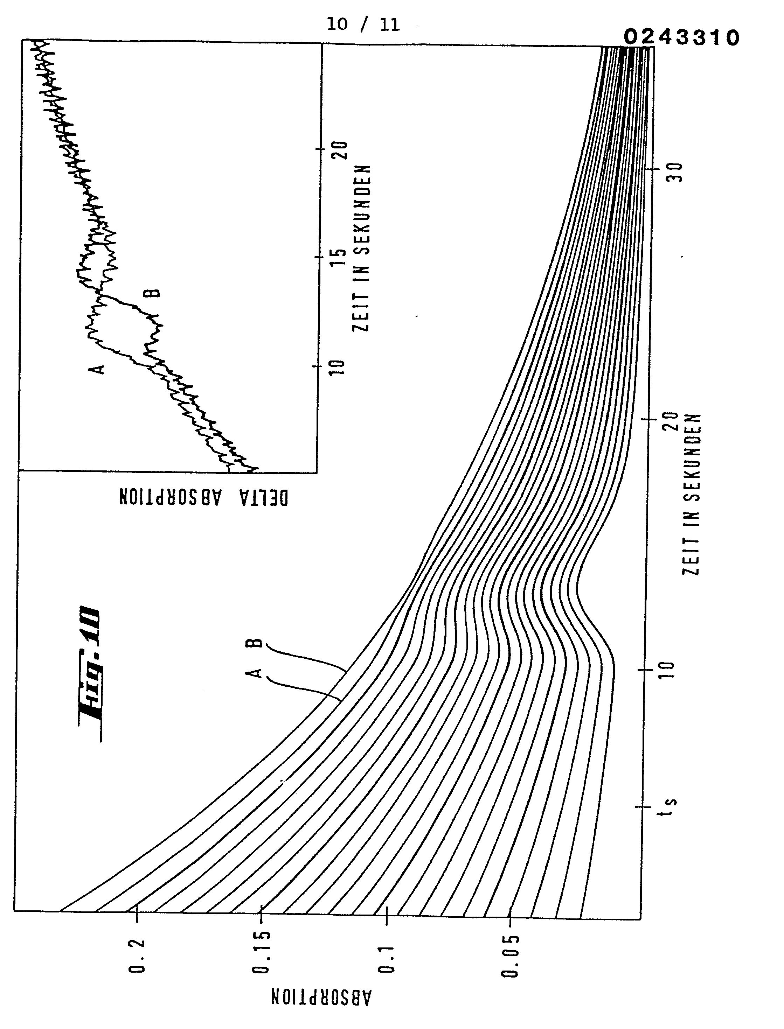

Figur 10 zeigt für den Fall der umgekehrten Strömungs-Injektions-Analyse das Eintauchen des induzierten Signals in das Hintergrundsignal eines Azofarbstoffes, wobei A und B den Phasenwechsel darstellen.

Figur 11 zeigt schematisch die Methode der Gradientenverdünnung kombiniert mit zeitlich variablem Einfangen einer Probe, wobei die sich ständig ändernde Konzentration durch verschiedene Gradienten dargestellt ist und wobei $t_1$ und $t_2$ ein Segment des Gradienten symbolisieren, welches über ein Ventil in ein zweites Strömungs-Injektions-Analysen-System eingespeist wird.

Die folgenden Beispiele dienen zur Erläuterung der Erfindung.

Darin sind die Teile Gewichtsteile und die Prozente Gewichtsprozente. Die Temperaturen sind in Celsiusgraden angegeben. Die Beziehung zwischen Gewichtsteilen und Volumenteilen ist dieselbe wie diejenige zwischen Gramm und Kubikzentimeter.

Beispiel 1: Herstellung von 1-Amino-3-(2',3'-dibrompropionyl-amino)-benzol-6-sulfonsäure als Beispiel für Verfahrenssteuerung per Strömungs-Injektions-Analyse durch Messung und Auswertung der Höhe und der Breite eines spektrophotometrisch ermittelten Signals.

Verfahren: 1-Amino-3-(2',3'-dibrompropionylamino)-benzol-6-sulfonsäure wird hergestellt aus 1,3-Phenylendiamin-4-sulfonsäure durch Acylierung mit 2,3-Dibrompropionylchlorid. Das Reaktionsprodukt ist ein farbloses Zwischenprodukt, welches ohne weitere Aufarbeitung bzw. Reinigung als Ausgangsverbindung für weitere Verfahrensschritte in einem diskontinuierlichen Verfahren (batch-process) eingesetzt wird. Es gilt deshalb den optimalen Zeitpunkt für den Reaktionsabbruch zu finden, da sowohl zu hohe Konzentration an Ausgangsmaterial wie zu hohe Konzentration an Nebenprodukt (zweifach acyliertes Phenylendiamin) zu Qualitätsproblemen führen.

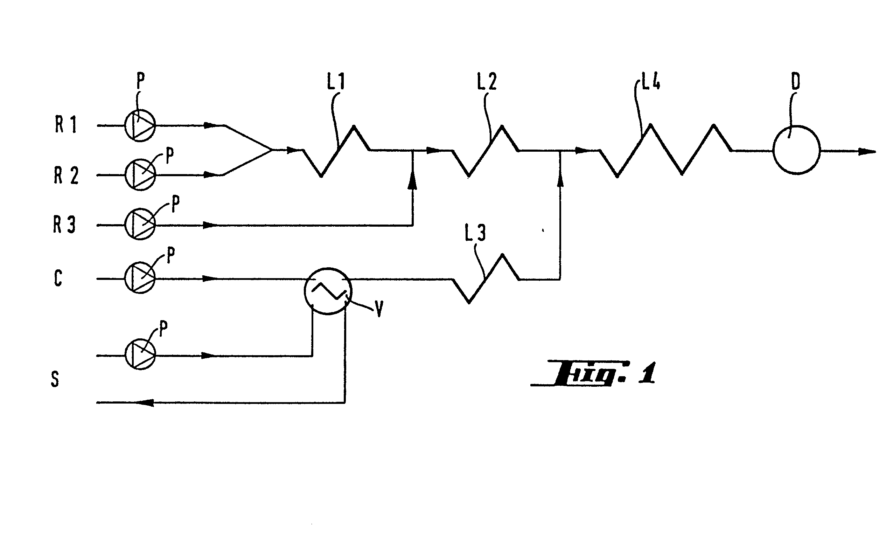

Strömungs-Injektions-Analysator: Eine reproduzierbare Qualität des oben angegebenen Verfahrensproduktes wird durch on-line Prozesssteuerung gewährleistet. Als analytische Methode im Analysator wird eine Azokupplungsreaktion durchgeführt. Der Aufbau des Analysators entspricht dem in Figur 1 dargestellten Aufbau, wobei

R1 ein Reservoir mit wässriger Natriumnitrit-Lösung (0,002 Mol/l)

R2 ein Reservoir mit p-Nitroanilin (0,002 Mol/l) mit ca. 0,5 % Schwefelsäure,

R3 ein Reservoir mit einer 1%-igen Lösung von Amidosulfonsäure in Wasser,

C  eine auf pH 4,5 gestellte wässrige Lösung, enthaltend 6 % Essigsäure, 13,6 % Natriumacetat, 4 % Natriumchlorid,

S  ein kontinuierlicher Probenstrom aus dem Reaktionskessel über eine by-pass-Leitung,

P  je eine Pumpe,

L1, L2, L3 und L4 je eine Rohrleitung zur Vermischung der Reagenzienströme,

V  ein Einspritzventil und

D  ein Durchflussphotometer ist.

Als Probenahme-System wird eines mit kontinuierlicher Filtration verwendet, wodurch das Produkt welches in der Reaktionsmasse als Feststoff vorliegt, nahezu vollständig abgetrennt wird.

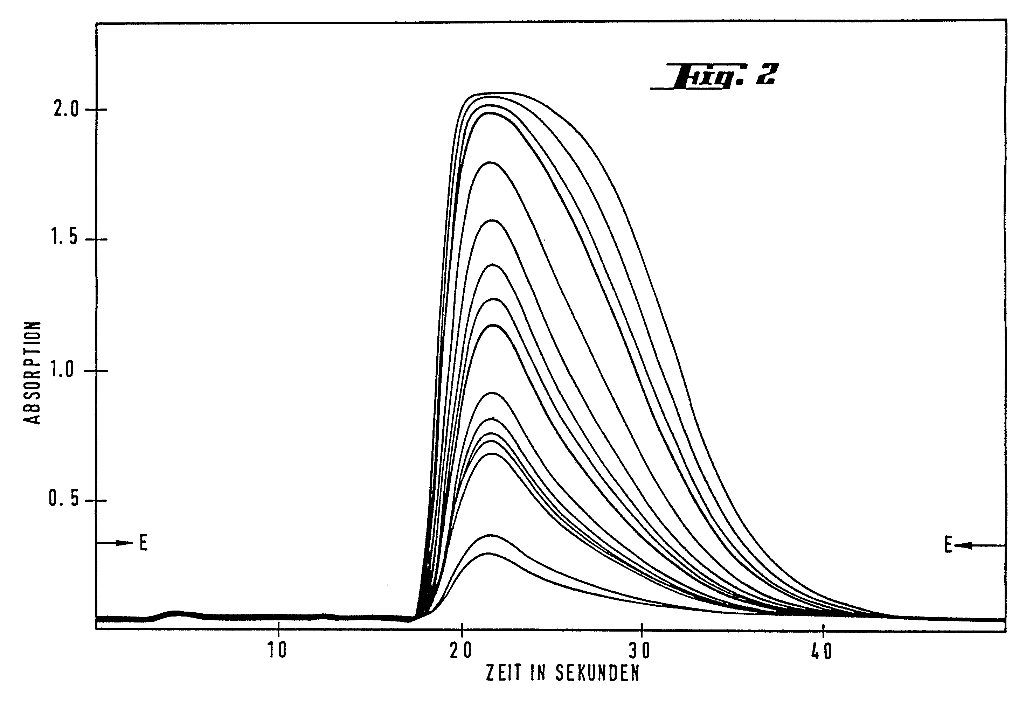

Im Analysator wird kontinuierlich das thermisch unstabile Diazoniumion des p-Nitroanilins aus dem Amin in salzsaurer Lösung und der wässrigen Natriumnitrit-Lösung hergestellt. Der Zusatz von Amidosulfonsäure ist notwendig, um überschüssiges Nitrit zu zerstören. Im Durchflussphotometer wird das Umsetzungsprodukt aus dem gebildeten Diazoniumion und der eingespritzten Probe registriert als sichtbare Absorption des in Gegenwart des Eduktes gebildeten Azofarbstoffes. Die ausgewählte Reagenzkonzentration sättigt den Analysator bei einer Absorption von ungefähr 2. Eine durch Adsorption des Farbstoffes an den Rohren oder dem Fenster der Durchflussküvette enstandene Verunreinigung wird durch die Carrier-Lösung verhindert, um eine stabile Basislinie beizubehalten. Der Analysator zeigt lineares Ansprechverhalten auf Edukt-Konzentrationen bis zu 700 ppm.
Darüber hinaus geht das Signal in Sättigung über. Figur 2 zeigt den
Verlauf der Reaktion bis zum Endpunkt. Figur 2 zeigt eine Folge von
Signalen registriert nach Zusatz von ca. 90 % des Acylierungsmittels
bis zum vorherbestimmten Endpunkt (E). Die Genauigkeit der Signalhöhen-Messung ist mit ca. 1 % relativer Standardabweichung genügend,
und die Genauigkeit der Signalbreiten-Messung ist mit ca. 2 %
relativer Standardabweichung akzeptabel, um die Anfangskonzentration
an Edukt sowie die Eduktkonzentration nach Zugabe von ca. 90 % des
Acylierungsmittels zu bestimmen. Die Prozesssteuerung zum vorgegebenen Qualitäts-Endpunkt (E) erfolgt nach der Zugabe von ca.
90 % des Acylierungsmittels durch kontinuierliche Signalhöhen-
Messung.

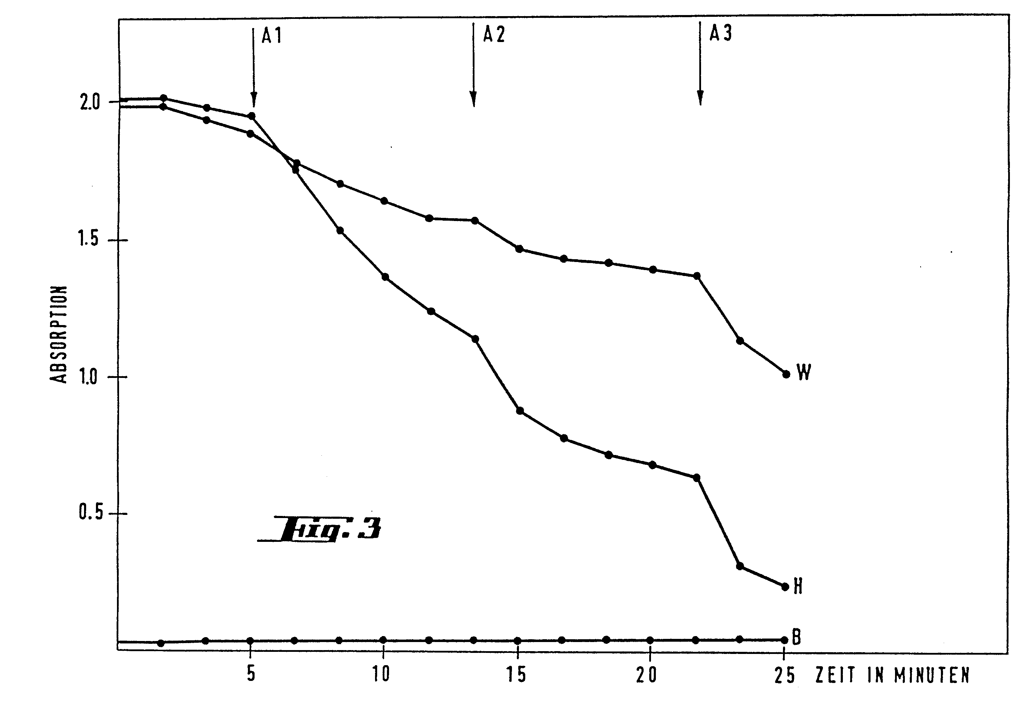

Figur 3 zeigt die Werte für gleichzeitige Signalhöhen- (H) und
Signalbreiten- (W) Messung des Azofarbstoffes nach Zugabe von
Acylierungsmittel (A1, A2 und A3) zur Steuerung des Verfahrens auf
den Endpunkt hin. Die Stabilität der Basislinie zeigt (B). Figur 3
ist representativ für die Antwort eines Analysators auf dreimaligen
Zusatz von Acylierungsmittel zu der Reaktionsmasse nahe beim
Endpunkt der Umsetzung. Für die Signalbreiten-Messung werden die
Werte einer Zeit-Achse auf Durchlässigkeitswerte umskaliert, um
beide Messungen (H) und (W) in einer Abbildung darzustellen.

Die im Detektor gemessenen Daten werden in einem Computer erfasst,
gespeichert und zur weiteren Prozesssteuerung verwendet.

Mit der oben angegebenen Auswertung der Daten der Signalbreiten-
Messung können Konzentrationen bis 20000 ppm gemessen werden, ohne
das Strömungs-Injektions-System zu ändern, d.h. durch Kombination
der Messmethoden, Signalhöhen-Messung und Signalbreiten-Messung kann
ein grosser Konzentrationsbereich abgedeckt werden.

Die Signalbreite ist annähernd proportional zum Logarithmus der
Konzentration der Probe. Die Signalbreite kann auf beliebiger Höhe
über der Basislinie gemessen werden. In der Nähe der Basislinie und in der Nähe des Signalmaximums werden wegen des Rauschens bzw. wegen der geringen Signalbreite weniger präzise Daten erhalten als im Bereich zwischen diesen Extremen.

Wird die Rohrleitung L4 in Figur 1 durch eine in Beispiel 2 angegebene Mischkammer ersetzt, wobei Mischkammer und Rohrleitung L4 identisches Totvolumen haben, erhält man ein etwas anderes Dispersionsprofil. Dadurch wird die Eichgerade der Signalbreiten-Messung etwas steiler und die Präzision somit etwas besser.

Anstelle der Auswertung der Signalbreite kann auch die sogenannte elektronische Verdünnung [electronic dilution] zur Erweiterung des dynamischen Messbereichs angewendet werden. Zusätzlich zum Signalmaximum misst man bei dieser Methode Extinctionswerte an verschiedenen Orten in der absteigenden Flanke des Gradienten. Jeder dieser Messorte deckt einen limitierten Konzentrationsbereich der Probe mit einer linearen Eichgeraden ab. Die Präzision dieser Messung ist am Signalmaximum am grössten und nimmt bei Messungen in der absteigenden Flanke des Gradienten mit zunehmender Entfernung vom Signalmaximum ab.

Analog zu dem oben angegebenen Beispiel der Steuerung eines Verfahrens auf den Endpunkt hin, lässt sich die Diazotierung eines Amins überwachen und steuern. Die gemäss Beispiel 1 mit 2,3-Dibrompropionylchlorid acylierte 1,3-Phenylendiamin-4-sulfonsäure wird nach Diazotierung mit Natriumnitrit in salzsaurer Lösung auf eine Kupplungskomponente, wie z.B. N,N-Diäthylanilin, 3-Methyl-N,N-diäthylanilin oder 1-(3'-Chlorphenyl)-3-methylpyrazol-5-on, gekuppelt. Die Kontrolle der Diazotierung erfolgt über die Erfassung der Distickstofftrioxid-Konzentration. Bei der Diazotierung, die mit Natriumnitrit und Salzsäure in Gegenwart eines Amins vorgenommen wird, bildet sich über die Stufe der salpetrigen Säure Distickstofftrioxid, welches mit dem Amin zu der Diazonium-Verbindung umgesetzt wird. Bei der Diazotierung wird man über die Intensität des registrierten Signals zweckmässigerweise die Nitrit-oder Aminzufuhr steuern, d.h. wenn das Signal einen durch Vorversuche ermittelten Sollwert erreicht hat, wird die Nitrit- oder Aminzufuhr entsprechend
korrigiert. Auf diese Weise kann man eine Ueberdossierung an Nitrit
vermeiden und auch während der Reaktion die Nitritzufuhr so steuern,
dass es zu keinen unerwünschten Nebenprodukten kommt.

Eine reproduzierbare Qualität des Verfahrensproduktes, Diazoniumsalz
der 1-Amino-3-(2',3'-dibrompropionylamino)-benzol-6-sulfonsäure,
wird durch on-line Prozesssteuerung gewährleistet. Als analytische
Methode im Analysator wird eine Azokupplungsreaktion durchgeführt.
Der Analysator zeigt folgenden Aufbau:
Ueber eine by-pass-Leitung strömt kontinuierlich die Reaktionsmasse
an einem Ventil vorbei, welches in zeitlich fixierten Abständen die
Reaktionsmasse in eine Carrier-Lösung (Salzsäure-Lösung) einspritzt.
Diese Carrier-Lösung transportiert die Reaktionsmasse zu einer
Gasdiffusionszelle, z.B. mit Teflon-Membran, so dass durch die
Teflon-Membran der Gasdiffusionzelle $N_2O_3$ in eine weitere Carrier-
Lösung (0,03 % Sulfanilsäure in 0,1 N Salzsäure) diffundiert. Durch
$N_2O_3$ erfolgt Diazotierung eines Teiles der Sulfanilsäure. In diesen
Strom der teilweise diazotierten Sulfanilsäure wird eine 0,03 %-ige
wässrige Lösung von 1-Phenyl-3-methylpyrazol-5-on, die mit Borax
auf den pH-Wert 10 gestellt ist, in den Carrier-Strom eingespeist.

Der resultierende Azofarbstoff wird in einem Durchflussphotometer
bei der Wellenlänge 430 nm registriert. Je nach Konzentration des in
der Carrier-Lösung vorhandenen $N_2O_3$, d.h. je nach Verfahrensstand
der Diazotierung in der Reaktionsmasse, ändert sich die Durchlässigkeit bzw. Extinction. Ein plötzlicher Anstieg der registrierten
Signalhöhe zeigt das baldige Ende der Diazotierung an.

Die im Detektor gemessenen Werte werden in einem Computer erfasst,
gespeichert und zur Prozesssteuerung verwendet.

Beispiel 2: Herstellung von Dinitrostilbendisulfonsäure als Beispiel
für Verfahrenssteuerung per Strömungs-Injektions-Analyse Verfahren: Dinitrostilbendisulfonsäure wird hergestellt aus p-Nitrotoluolsulfonsäure durch Oxidation bei 90°C mit Natriumhypochlorit in stark alkalischem Medium. Dabei treten farblose und farbige Zwischen- und Nebenprodukte auf. Ihre Menge wird bestimmt durch die Reaktionsbedingungen und die Reaktionszeit, da das Produkt unter den Reaktionsbedingungen nicht stabil ist. Es gilt deshalb, den optimalen Zeitpunkt zu finden, bei dem möglichst viel Edukt und Zwischenprodukt verbraucht ist, aber noch möglichst wenig Produkt zerstört ist. Das elektrochemische Potential der Reaktionsmasse muss während der Umsetzung konstant gehalten werden, um hohe Ausbeute und gute Produktqualität zu gewährleisten. Dies wird erreicht durch ständige Kontrolle des elektrochemischen Potentials, wobei Abweichungen durch Zugabe von Natriumhypochlorit korrigiert werden. Das elektrochemische Potential der Reaktionsmasse wird während der gesamten Umsetzung mit einer Elektrode gemessen.

Eine zu geringe Menge an Oxidationsmittel induziert die Bildung von gelben Nebenprodukten, die bei 450 nm absorbieren, während die normale Menge an Oxidationsmittel, insbesondere ein Ueberschuss an Oxidationsmittel, das Produkt zerstört (Absorption des Produktes bei 353 nm) zu Nebenprodukten, die in einem Spektralbereich absorbieren, der mit dem Bereich des Eduktes (Maximum bei 280 nm) überlappt.

Strömungs-Injektions-Analysator: Der Aufbau des Analysators entspricht dem in Figur 4 gezeigten Aufbau, wobei

C   ein wässriger Carrier-Strom,

S   der 90°C heisse Probenstrom vom Probennahmesystem kommend,

P   je eine Pumpe,

V1  Einspritzventil,

V2  Einspritzventil, um einen Gradienten der Probenzone zu selektieren und einzufangen,

G   eine magnetisch gerührte Mischkammer

D   ein Durchflussphotometer ist.

Der in Figur 4 gezeigte Strömungs-Injektions-Analysator, der zur Verhinderung von Kristallisation im Probenkreislauf hohe Temperatur und hohe Flussrate benötigt, besteht im Prinzip nur aus einem Teil zur Probenvorbereitung in Kombination mit dem Detektor. Durch
Einschalten des Injektionsventils (V2 in Figur 4) können kleine
Volumina (z.B. 20 µl) der verdünnten Probenzone in der Durchflusszelle stationär gehalten werden. Die Mischkammer mit einer Grösse
von 500 µl dient hauptsächlich der Verhinderung der Produktkristallisation und der sofortigen Temperaturabsenkung der heissen
Probenzone, um die Reaktion zum Stillstand zu bringen. Ferner
erzeugt die Mischkammer einen Gradienten der eingespritzten Probe
für die basislinienkorrigierte spektrophotometrische Messung bei
zwei verschiedenen Verdünnungen und drei verschiedenen Spektralbereichen. Figur 5 zeigt die Anfangsphase des Verfahrens. Kurz vor der
Probeneinspritzung wird die Basislinien-Absorption bei 280 nm und
353 nm relativ zu der Absorption bei 450 nm bestimmt (B in Figur 5).
Der Konzentrationsgradient der Probe, der die Mischkammer verlässt
wird bei 450 nm registriert und das Maximum bestimmt. Bei einem
Verdünnungsfaktor von ca. 1500 werden die basislinienkorrigierte
Absorption der "gefangenen" Probenzone (V2 in Figur 5; 20 µl) bei
353 und 280 nm gemessen.
Figur 6 zeigt die Endphase des Verfahrens.

Bei einer Einspritzfrequenz von 30 Proben pro Stunde einschliesslich
der Probennahme werden 90 Einzelinformationen pro Stunde erhalten.
Diese Zahl an Daten ist ausreichend, um das Verfahren optimal zu
steuern. Die Resultate der synchronen spektrophotometrischen
Kontrolle der Edukt-Abnahme, der Produkt-Bildung und der Bildung von
Nebenprodukten werden kombiniert mit den Resultaten der weniger
selektiven elektrochemischen Messung zur Prognose (feed-forward
Kontrolle) der Oxidationsmittel-Zugabe, wodurch Ausbeute und
Qualität des Produktes optimiert wird.

Da die Analyse bei drei ausgewählten Wellenlängen bezüglich Verfahrenszeitpunkt und Ort der Probennahme von einer identischen Probe
stammt, ist jedes Verhältnis der Absorption an verschiedenen
Wellenlängen unabhängig vom Volumen der tatsächlichen Reaktionsmasse. Eine Eichung des Systems ist überflüssig, da nur relative Aenderungen der Werte der Absorption als relevante Parameter zur Prozesskontrolle verwendet werden.

Figur 7 zeigt die für die oben genannte chemische Reaktion gemessenen zeitabhängigen basislinienkorrigierten Absorptionswerte während der gesamten Umsetzung. Die gemessenen Werte bei 280 nm zeigen den Edukt-Verbrauch, die gemessenen Werte bei 353 nm zeigen die Produkt-Bildung, und die gemessenen Werte bei 450 nm zeigen die Bildung der Nebenprodukte, wobei die letzteren zur Darstellung in der Figur 7 mit dem Faktor 5 reduziert wurden.

Beispiel 3: Verfahren zur Herstellung des Azofarbstoffes aus diazotiertem p-Nitroanilin und H-Säure (1-Amino-8-hydroxynaphthalin-3,6-disulfonsäure) mittels Steuerung durch umgekehrte Strömungs-Injektionsanalyse [reversed flow injection analysis].

Verfahren: Ein Reaktionskessel wird mit 1-Amino-8-hydroxynaphthalin-3,6-disulfonsäure in wässriger Lösung (pH 10) beladen. Zu dieser Lösung lässt man kontinuierlich die wässrige Lösung des Diazoniumsalzes des p-Nitroanilins zulaufen. Nach kurzer Reaktionszeit wird ein Azofarbstoff erhalten, der photometrisch im sichtbaren Bereich des Spektrums mit genügender spektroskopischer Selektivität absorbiert. Die Reaktionslösung wird ständig mit hoher Geschwindigkeit aus dem Kessel durch das Einspritzventil des Analysators gepumpt.

Strömungs-Injektions-Analysator: Der Aufbau des Analysators entspricht dem in Figur 8 gezeigten Aufbau, wobei

R der Reagenzstrom, enthaltend ca. 0,005 Mol/l des Diazoniumsalzes des p-Nitroanilins in wässriger Lösung,

C wässriger Carrier-Strom, enthaltend einen Phosphatpuffer zur pH-Einstellung von 8,

S Probenstrom vom Probennahmesystem,

P je eine Pumpe,

V1 Probeneinspritzventil,

V2 Reagenzeinspritzventil,

G eine magnetisch gerührte Mischkammer,

L eine Säule

D ein Durchflussphotometer ist.


Eine Probe aus dem Reaktionskessel wird kontinuierlich in Intervallen von 150 Sekunden in den Analysator eingespritzt und sofort mit der Pufferlösung in der Mischkammer vermischt. Die verlängerte und vorbehandelte Probenzone fliesst aus der Mischkammer durch das Ventil V2. Zu dieser Lösung wird eine Lösung des gleichen Diazoniumsalzes wie in dem Reaktionskessel in den Gradienten bei einer Verdünnung von ca. 3000 eingespritzt. Die Höhe des resultierenden Signals welches auf dem abfallenden Ende des gefärbten Probengradienten aufgezeichnet wird, hängt ab von der Konzentration der Kupplungskomponente im Reaktionsgefäss zur Zeit der Probennahme. Um eine ausreichende Empfindlichkeit unter den Bedingungen der umgekehrten Strömungs-Injektion-Analyse zu erhalten, wird der Analysator im Bereich der Konzentration der Kupplungskomponente in der Nähe des Endpunktes der Reaktion optimiert. Während des Verfahrens steigt die Hintergrund-Absorption entsprechend der Bildung des Azofarbstoffes an. Figur 9 zeigt die Reproduktion von vier umgekehrten Strömungs-Injektionen in den Gradienten der Probe bei einem Verdünnungskoeffizienten von ca. 3000. Die Wiederholbarkeit ist gegeben. Der Analysator liefert folgende prozesstechnisch relevanten Informationen:


1. Die Hintergrundabsorption des verdünnten Gradienten, gemessen an einer willkürlich ausgewählten Zeit $t_s$, steht in direkter Beziehung zu der Konzentration des im Verlauf des Verfahrens gebildeten Azofarbstoffes. Eine hohe Analysenfrequenz hinsichtlich der gesamten Verfahrenszeit, ermöglicht eine Prognose für den Endpunkt des Verfahrens nach jeder einzelnen Analyse.


2. Aus jeder einzelnen eingespritzten Probe ist eine Prognose für den Endpunkt des Verfahrens möglich über den Verbrauch an Kupplungskomponente, welcher aus der Signalhöhe des im Analysator induzierten Signals resultiert.

3. Das Eintauchen des induzierten Signals in das Hintergrundsignal des Azofarbstoffes geschieht zu einem ganz bestimmten Zeitpunkt des Verfahrens, vgl. A und B in Figur 10. Dieser Verfahrenspunkt ist als Phasenwechsel der ersten Ableitung des Signals feststellbar, vgl. Einschub in Figur 10. Der Verfahrenszeitpunkt an dem dieser Phasenwechsel auftritt, entspricht einer ganz bestimmten Restkonzentration an unumgesetzter Kupplungskomponente. Diese Restkonzentration kann nach anderen Analysemethoden (z.B. chromatographisch) festgestellt werden. Unter der Voraussetzung, dass die Umsetzung bei jedem Neubeginn dieses Prozesses reproduzierbar unter physikalischer und chemischer Kontrolle gehalten wird, stellt der Zeitpunkt für den Phasenwechsel einen definierten Verfahrenszustand hinsichtlich Produktbildung und Eduktverbrauch dar, wodurch die Steuerung des Verfahrens zum Optimum möglich ist.

Die in dem oben angegebenen Beispiel durchgeführte Prozesssteuerung ist allgemein anwendbar auf Verfahren, die eine starke Störung im Hintergrund z.B. die Absorption eines im Verlauf des Verfahrens gebildeten Azofarbstoffes haben.

In den Figuren 2, 3, 5, 6, 7, 9, 10 und 11 bedeutet Absorption die Durchlässigkeit bzw. Extinction.

Patentansprüche

1. Verfahren zur Steuerung und Optimierung chemischer, industrieller Prozesse, dadurch gekennzeichnet, dass man die Methode der Strömungs-Injektions-Analyse zur Steuerung und Optimierung von Herstellungsverfahren von Textilveredlungsmitteln und Textilausrüstmitteln und deren Zwischenprodukten anwendet.

2. Verfahren gemäss Anspruch 1, dadurch gekennzeichnet, dass man die Methode der Strömungs-Injektions-Analyse zur Steuerung und Optimierung diskontinuierlicher Herstellungsverfahren anwendet.

3. Verfahren gemäss einem der Ansprüche 1 und 2, dadurch gekennzeichnet, dass man Herstellungsverfahren für Farbstoffe, optische Aufheller und deren Zwischenprodukte steuert und optimiert.

4. Verfahren gemäss Anspruch 1, dadurch gekennzeichnet, dass man Ein-Phasen- oder insbesondere Multi-Phasen-Herstellungsverfahren steuert und optimiert.

5. Verfahren gemäss einem der Ansprüche 1 bis 4, dadurch gekennzeichnet, dass man die Methode der Strömungs-Injektions-Analyse zur on-line Steuerung und Optimierung verwendet.

6. Verfahren gemäss einem der Ansprüche 1 bis 5, dadurch gekennzeichnet, dass man Edukt- und/oder Produkt-Konzentrationen zur Steuerung und Optimierung verwendet.

7. Verfahren gemäss einem der Ansprüche 1 bis 5, dadurch gekennzeichnet, dass man Nebenprodukt-Konzentrationen zur Steuerung und Optimierung verwendet.

8. Verfahren gemäss einem der Ansprüche 1 bis 7, dadurch gekennzeichnet, dass man die Methode der Strömungs-Injektions-Analyse zur reproduzierbaren Festsetzung des Verfahrensendpunktes und der Produktequalität anwendet.

9. Verfahren gemäss einem der Ansprüche 1 bis 8, dadurch gekennzeichnet, dass man die Herstellungsverfahren durch Auswertung der Daten der Signalhöhen- und/oder der Signalbreiten-Messung steuert und optimiert.

10. Verfahren gemäss einem der Ansprüche 1 bis 8, dadurch gekennzeichnet, dass man die Herstellungsverfahren durch Auswertung der Daten der elektronischen Verdünnung steuert und optimiert.

11. Verfahren gemäss einem der Ansprüche 1 bis 8, dadurch gekennzeichnet, dass man die Herstellungsverfahren durch Auswertung der Daten der Gradienten-Verdünnung mit zeitlich variablem zone-sampling steuert und optimiert.

12. Verfahren gemäss einem der Ansprüche 1 bis 11, dadurch gekennzeichnet, dass man eine gerührte Mischkammer zur Ausbildung des Konzentrationsgradienten im Strömungs-Injektions-Analysator verwendet.

13. Verfahren gemäss einem der Ansprüche 1 bis 12, dadurch gekennzeichnet, dass man die Methode der Strömungs-Injektions-Analyse zur Steuerung und Optimierung von Acylierungsreaktionen verwendet.

14. Verfahren gemäss einem der Ansprüche 1 bis 12, dadurch gekennzeichnet, dass man die Methode der Strömungs-Injektions-Analyse zur Steuerung und Optimierung von Diazotierungen verwendet.

15. Verfahren gemäss Anspruch 14, dadurch gekennzeichnet, dass man zur Steuerung und Optimierung von Diazotierungsreaktionen eine Gasdiffusionsmembran als Probenahmesystem verwendet.

16. Verfahren gemäss einem der Ansprüche 1 bis 12, dadurch gekennzeichent, dass man die Methode der Strömungs-Injektions-Analyse zur Steuerung und Optimierung von Kupplungsreaktionen verwendet.

17. Verfahren gemäss einem der Ansprüche 1 bis 13, dadurch gekennzeichnet, dass man im Strömungs-Injektions-Analysator eine Probe, die mittels kontinuierlicher Filtration, Homogenisierung oder Verdünnung mit einem Lösungsmittel oder mittels einer Membran konditioniert wurde, verwendet.

18. Verfahren gemäss den angegebenen Beispielen.

19. Anwendung des Verfahrens gemäss Anspruch 1 zur Konzentrationsbestimmung an Edukt und/oder Produkt bei Herstellungsverfahren gemäss Anspruch 1.

20. Anwendung des Verfahrens gemäss Anspruch 1 zur Analyse des Produkt/Edukt-Verhältnisses und zur Erfassung von Nebenprodukten bei der Herstellung von Farbstoffen, optischen Aufhellern und deren Zwischenprodukten.

21. Anwendung des Verfahrens gemäss Anspruch 1 zur on-line-Steuerung eines computer-integrierten, automatisierten Herstellungsverfahrens.

22. Anwendung des Verfahrens gemäss Anspruch 1 zur on-line-Steuerung eines computer-integrierten, automatisierten Herstellungsverfahrens für Farbstoffe, optische Aufheller und deren Zwischenprodukte.

FO 7.1/JZ/we*/ch*

Fig. 1

Fig. 2

ABSORPTION

2.0

1.5

1.0

0.5

E

E

10    20    30    40

ZEIT IN SEKUNDEN

2 / 11

0243310

Fig.3

*Fig. 4.*

V2

D

G

V1

P

P

C

S

Fig. 5

ABSORPTION

0.5  1.0  1.5  2.0

ZEIT IN SEKUNDEN

10  20  30  40

B

450 nm

353 nm

280 nm

Fig. 6

Fig. 7

Fig. 8

0243310

**Fig. 9**

ABSORPTION

ZEIT IN SEKUNDEN

Fig. 10

ABSORPTION

0.2
0.15
0.1
0.05

A B

t$_s$    10    20    30

ZEIT IN SEKUNDEN

DELTA ABSORPTION

A
B

10    15    20

ZEIT IN SEKUNDEN

10 / 11

0243310

Fig. 11